Europäisches Patentamt

European Patent Office

Office européen des brevets

(19)

(11) Veröffentlichungsnummer: **0 063 222**
**B2**

(12) **NEUE EUROPÄISCHE PATENTSCHRIFT**

(45) Veröffentlichungstag der neuen Patentschrift:
**09.01.91**

(21) Anmeldenummer: **82101472.7**

(22) Anmeldetag: **26.02.82**

(51) Int. Cl.[5]: **A 61 K 7/16**

(54) Zahnpflegemittel.

(30) Priorität: **10.04.81 DE 3114492**

(43) Veröffentlichungstag der Anmeldung:
**27.10.82 Patentblatt 82/43**

(45) Bekanntmachung des Hinweises auf die
Patenterteilung:
**30.12.86 Patenblatt 86/52**

(45) Bekanntmachung des Hinweises auf die
Entscheidung über den Einspruch:
**09.01.91 Patentblatt 91/02**

(84) Benannte Vertragsstaaten:
**AT BE CH DE FR GB IT LI NL SE**

(56) Entgegenhaltungen:
**DE-A-2 206 285**
**FR-A-2 272 033**
**US-A-4 122 160**

**Horak et al, Powder Technology, vol. 31, 1982;**
**S. 263-267**

**Die Akte enthält technische Angaben, die nach**
**dem Eingang der Anmeldung eingereicht**
**wurden und die nicht in dieser Patentschrift**
**enthalten sind.**

(73) Patentinhaber: **Degussa Aktiengesellschaft**
**Weissfrauenstrasse 9**
**D-6000 Frankfurt am Main 1 (DE)**

(72) Erfinder: **Müller, Karl-Hans, Dr.**
**Robert Koch-Str. 17**
**D-6434 Bruchköbel (DE)**
Erfinder: **Neumüller, Matthias**
**Lindenstr. 3**
**D-6467 Hasselroth 1 (DE)**
Erfinder: **Türk, Günter, Dr.**
**Liesingstr. 3**
**D-6450 Hanau 9 (DE)**

Courier Press, Leamington Spa, England.

## Beschreibung

Es ist bekannt, amorphe synthetische Kieselsäuren als Putzkörper in Zahnpasten zu verwenden. Kieselsäuren haben als Putzkörper im Vergleich zu den bisher verwendeten Calziumphosphaten und Kreiden den entscheidenden Vorteil, daß sie mit den häufig in Form von Zinn (II)-fluorid und Natriumfluorid als Anticariesmittel eingesetzten Fluoridionen nicht reagieren und somit die Wirkung dieser Zusätze nicht blockieren.

Es besteht also ein Bedürfnis, die auf Calziumphosphaten und Kreiden basierenden Putzkörper durch einen inerten Stoff zu ersetzen.

Ein erster entscheidender Schritt in dieser Richtung gelang mit der Einführung von amorphen hochporösen Kieselsäurexerogelen (US—PS 2,538,230), deren Kombination mit verdickend wirkenden Aerogelen — wie z.B. Syloid 244® — der Verwendung von Kieselsäuregelen in Zahnpasten endgültig zum Durchbruch verhalf. Dabei muß allerdings darauf hingewiesen werden, daß bereits lange vorher von der viskositätsregulierenden Wirkung bestimmter Kieselsäuren, wie z.B. der pyrogenen und der Fällungskieselsäuren mit hoher Struktur Gebrauch gemacht wurde (DE—PS 1 667 875). In der fortschreitenden Entwicklung wurde dann auch konsequenterweise die Kombination von Putzkörpern auf der Basis von Kieselsäure-Xerogelen mit pyrogenen Kieselsäuren (DE—AS 2 033 678) und mit verdickend wirkenden mittel- bis hochstrukturierten Fällungskieselsäuren (GB—PS 1 433 743, DE—AS 2 610 207, und US—PS 4,132,806) aufgenommen.

Der nächste Schritt im Hinblick auf die Einführung verbesserter neuer Putzkörper war die Entwicklung eines speziellen Aluminiumsilikates auf dem Fällungswege, dessen Kombination mit einer verdickend wirkenden pyrogenen Kieselsäure, bzw. mit einer verdickend wirkenden mittel- bis hochstrukturierten Fällungskieselsäure in den Patentschriften DE—PS 2 154 376 und DE—AS 2 206 285 beschrieben wird.

Die weitere Entwicklung führte dann zu Putzkörpern auf der Basis von Fällungskieselsäuren ohne nennenswerte Verdickungswirkung, jedoch mit einer im Vergleich zu den Calziumphosphat-Putzkörpern deutlich herabgesetzten Abrasivität. Im Falle der Verwendung dieser neuen $SiO_2$-basierenden Fällungskieselsäure erfolgt die Regulierung der Rheologie der Zahnpasten nicht mittels verdickend wirkender Fällungskieselsäuren, sondern mittels spezieller Bindemittelzusätze wie Seetang-Kolloiden, Synthetischen Cellulosederivaten (Carragheen und Natriumcarboxymethylcellulose) und mittels Planzengummen. (DE—OS 2 344 316; US—PS 4,105,757; DE—OS 2 344 805; US—PS 4,122,160; US—PS 4,144,321 und DE—OS 2 522 486).

Neuerdings werden an die Wirkungen eines fortschrittlichen Putzkörpers auf der Basis von Kieselsäuren in Zahnpasten folgende Anforderungen gestellt:

— gemilderte Abrasionswirkung im Sinne einer mittleren Polierwirksamkeit (Cu-Abrieb in der fertigen Pasta zwischen 8—14 mg) zum Schutze des Zahnschmelzes,

— bei Füllungsgraden im Bereich von 15—25 Gew.-% bezogen auf die Menge ein- und desselben bifunktionellen Putzkörpers (Abrasion und Verdickung) sollen sich bei obiger Cu-Abriebspanne Viskositäten zwischen 2.500—4.500 mPas (Methode Rotovisko-PK) einstellen.

— Ausbildung eines glatten, glänzenden, strippenfreien Zahnpastenstranges und

— verbesserte Lagerfähigkeit des den Putzkörper enthaltenden Zahnpflegemittels in Hinblick auf eine gleichbleibende Abrasivität und Verdickung (keine Nachverdickung!) für die Dauer von mindestens einem Jahr.

Der Erfindung lag die Aufgabenstellung zugrunde, durch die Auswahl und Verwendung eines geeigneten Putzkörpers die obigen an ein Zahnpflegemittel gestellten Anforderungen zu erfüllen.

Zur Lösung dieser Aufgabe kann wie folgt verfahren werden:

Es wird auf ein Verfahren zur Herstellung von Fällungskieselsäuren zurückgegriffen, das je nach Art ihrer Vermahlung zu Fällungskieselsäuren mit folgenden physikalisch-chemischen Kenndaten führt:

|  | Pendelmühle | Strahlmühle |
|---|---|---|
| BET-Oberfläche nach<br>DIN 66 131 (m²/g) | 15—110 | 15—110 |
| Stampfgewicht nach<br>DIN 53 194 (g/l) | 150—750 | 90—650 |
| Cu-Abrieb in 10%iger<br>Gylserin-Dispersion (mg) | 5—30 | 5—30 |
| Viskosität in 30%iger<br>Glycerin-Wasser-Dispersion<br>(1:1), Brookfield (mPas) | 30000—60000 | 30000—60000 |
| RTV Sp 5<br>Hellbezugswert A nach<br>DIN 55 921 (%) | 86—96 | 90—96 |
| Sekundärteilchengrößen-<br>verteilungskurfe nach<br>Coulter Counter | gem. Fig. 1 | gem. Fig. 2 |
| "ALPINE"-Siebrückstand<br>>63 µm (Gew.-%) | <1,5 | <0,1 |

Derartige Fällungskieselsäuren eignen sich ganz besonders für die Verwendung in den erfindungsgemäßen Zahnpflegemitteln, die das oben genannte Eigenschaftsbild aufweisen sollen.

Das Verfahren zur Herstellung von Fällungskieselsäuren mit dem obigen Kenndatenbild zeichnet sich dadurch aus, daß man eine Originalfällungskieselsäuresuspension, die durch Fällung der Kieselsäure in einer vorgelegten Alkalisilikatlösung mit einer Konzentration von etwa 5—25 g SiO₂ je Liter Lösung mit einer Säure- und Alkalimetallsilikatlösung mit bestimmter Lösungskonzentrationen und bestimmten Zulaufgeschwindigkeiten unter Aufrechterhaltung einer Fälltemperatur im Reaktionsmedium zwischen 80 und 90°C derart hergestellt wird, daß die Viskosität des Reaktionsmediums in einem Zeitraum von mindestens 30% der gesamten Fälldauer gleichmäßig niedrig und der pH-Wert zwischen 10 und 12 gehalten wird, und die Zugabe der Reaktionsteilnehmer erst dann beendet wird, wenn die Viskosität nach Durchlaufen eines Maximums auf einem weniger als 100% über der Anfangsviskosität liegenden Wertes gesunken ist, mit heißem Wasser auf einen Fällungskieselsäuregehalt von 10—30 g/l und Natrium-sulfatgehalt von 6—20 g/l verdünnt, auf 85—95°C erwärmt, den pH-Wert mit Schwefelsäure auf 7—9 einstellt und unter Konstanthalten dieses pH-Wertes durch gleichzeitigen Zulauf von Alkalimetallsilikatlösung, Schwefelsäure und heißem Wasser über eine Fälldauer von 30—180 Minuten eine Fällungskieselsäureendkonzentration von 40—80 g/l einstellt, die Suspension mit konzentrierter Schwefelsäure auf einen pH-Wert von unter 7 ansäuert, die Fällungskieselsäure mit Hilfe einer Filterpresse aus der Suspension abtrennt, auswäscht, trocknet und mittels einer Pendelmühle oder Strahlmühle vermahit.

In einer bevorzugten Ausführungsform kann eine Originalfällungskieselsäuresuspension verwendet werden, die während ihrer Herstellungsphase gemäß DE—AS 1 467 019 intensiv geschert wird. Das ist immer dann von Vorteil, wenn besonders hohe Cu-Abriebwerte erzielt werden sollen. In einer besonderen Ausführungsform der Erfindung kann man die Scherung gemäß der DE—PS 1 767 332 durchführen.

Gegenstand der Erfindung ist ein Zahnpflegemittel mit verbesserter Lagerstabilität im Hinblick auf Rheologie und Abrasivität, das dadurch gekennzeichnet ist, daß es als Verdickungs- und Abrasivmittel in einer Menge von 20 bis 25 Gew.-% eine gefällte Kieselsäure enthält, die durch folgende physikalische-chemischen Kenndaten charakterisiert wird:

| | |
|---|---|
| BET-Oberfläche nach DIN 66 131 | m²/g 15—110 |
| Stampfgewicht | g/l 90—650 |
| Sekundärteilchengrößen-Verteilungkurve nach Coulter Counter gem. Fig. 2 "ALPINE"-Siebrückstand >63 μm | Gew.-% <0,1 |
| Cu-Abrieb in 10%iger Glyzerin-dispersion | mg 5—30 |
| Viskosität in 30%iger Wasser-Glyzerindispersion (1:1) Brookfield RTV Sp 5 | mPas 30 000—60 000 |
| Hellbezugswert A nach DIN 55 921 | % 90—96 |

und dadurch erhältlich ist, daß man eine Originalfällungs-kieselsäuresuspension, die durch Fällung der Kieselsäure aus einer vorgelegten Alkalisilikatlösung mit einer Konzentration von etwa 5—25 g SiO₂ je Liter Lösung mit einer Säure- und Alkalisilikatlösung mit bestimmten Lösungskonzentrationen und bestimmten Zulaufgeschwindigkeiten unter Aufrechterhaltung einer Fälltemperatur, im Reaktionsmedium zwischen 80 und 90°C derart hergestellt wird, daß die Viskosität des Reaktionsmediums in einem Zeitraum von mindestens 30% der gesamten Fälldauer gleichmäßig niedrig und der pH-Wert zwischen 10 und 12 gehalten wird, und die Zugabe der Reaktionsteilnehmer erst dann beendet wird, wenn die Viskosität nach Druchlaufen eines Maximums auf einen weniger als 100% über der Anfangsviskosität liegenden Wert gesunken ist, mit heißem Wasser auf einen Fällungskieselsäuregehalt von 10—30 g/l und Natriumsulfat-gehalt von 6—20 g Na₂SO₄/l verdünnt, auf 85—95°C erwärmt, den pH-Wert mit Schwefelsäure auf 7 bis 9 einstellt und unter Konstanthalten dieses pH-Wertes durch gleichzeitigen Zulauf von Alkalisilikatlösung. Schwefelsäure und heißem Wasser über eine Fälldauer von 15 bis 180 Minuten eine Fällungskieselsäureendkonzentration von 40—80 g/l einstellt, die Suspension mit konzentrierter Schwefelsäure auf einen pH-Wert unter 7 ansäuert, die Fällungskieselsäure mit Hilfe einer Filterpresse aus der Suspension abstrennt, auswäscht, trocknet und mittels einer Strahlmühle vermahlt.

Das erfindungsgemäße Zahnpflegemittel weist die folgenden vorteilhaften Eigenschaften auf:

— milde Abrasitivät im Sinne einer mittleren Polierwirkung der Zahnoberfläche ohne Schädigung des Zahnschmeltes.

— bei Anwendung von 15—25 Gew.-% Putzkörper auf der Basis einer einzigen Kieselsäure lassen sich milde Abrasion (8—14 mg Cu-Abrieb) mit optimaler Rheologie der Paste (Viskosität zwischen 2 500 und 4 500 mPas Methode Rotovisko) erreichen.

— Ausbildung eines glatten, stippenfreien, glänzenden Zahnpastastranges und

— verbesserte Lagerfähigkeit im Hinblick auf eine gleichbleibende Abrasivität und Verdickung (ohne Nachverdickung!).

Ein weiterer Gegenstand der Erfindung ist ein Zahnpflegemittel mit verbesserter Lagerstabilität in Hinblock auf Rheologie und Abrasivität, das dadurch gekennzeichnet ist, daß es als Abrasivmittel in einer Menge von 20 bis 25 Gew.-% eine gefällte Kieselsäure enthält, die durch folgende physikalisch-chemische Kenndaten charakterisiert wird:

| | |
|---|---|
| BET-Oberfläche nach DIN 66 131 | m²/g 15—110 |
| Stampfgewicht | g/l 150—750 |
| Sekundärteilchengrößen-Verteilung gemäß Figur 1 "ALPINE"-Siebrückstand >63 μm | Gew.-% <1,5 |
| Cu-Abrieb in 10%iger Glyzerindispersion | mg 5—30 |
| Viskosität in 30%iger Glyzerin-Wasser-dispersion (1:1) Brookfield RTV Sp 5 | mPas 30 000—60,000 |
| Hellbezugswert A nach DIN 55 921 | % 86—96 |

und dadurch erhältlich ist, daß man eine Originalfällungkieselsäuresuspension, die durch Fällung der Kieselsäure aus einer vorgelegten Alkalisilikatlösung mit einer Konzentration von etwa 5—25 g SiO₂ je Liter Lösung mit einer Säure und Alkalimetallsilikatlösung mit bestimmten Lösungskonzentrationen und bestimmten Zulaufgeschwindigkeiten unter Aufrechterhaltung einer Fälltemperatur im Reaktionsmedium zwischen 80 und 90°C derart hergestellt wird, daß die Viskosität des Reaktionsmedium in einem Zeitraum von mindestens 30% der gesamten Fälldauer gleichmaßig niedrig und der pH-Wert zwischen 10 und 12 gehalten wird, und die Zugabe der Reaktionsteilnehmer erst dann beendet wird, wenn die Viskosität nach Duruchlaufen eines Maximums auf einem weniger als 100% über der Anfangsviskosität liegenden Wert gesunken ist, mit heißem Wasser auf einen Fällungskieselsäuregehalt von 10—30 g/l und Natriumsulfat-gehalt von 6—20 g Na₂SO₄/l verdünnt, auf 85—95°C erwärmt, den pH-Wert mit Schwefelsäure auf 7 und 9

4

einstellt und unter Konstanthalten dieses pH-Wertes durch gleichzeitigen Zulauf von Alkalimetallsilikatlösung, Schwefelsäure und gegebenenfalls heißem. Wasser über eine Fälldauer von 15 bis 180 Minuten eine Fällungskieselsäureendkonzentration von 40 bis 80 g/l einstellt, die Suspension mit konzentrierter Schwefelsäure auf einen pH-wert unter 7 ansäuert, die Fällungskieselsäure mit Hilfe einer Filterpresse aus der Suspension abtrennt, auswäscht, trocknet und mittels einer Pendelmühle vermahlt.

Wird die Abrasivkieselsäure als alleinige Komponente verwendet, liegt die Konzentration bei 20 bis 25 Gew.-% vorzugsweise 20 bis 22 Gew.-%.

Dieses erfindungsgemäße Zahnpflegemittel weist folgende vorteilhafte Eigenschaften auf:
— milde Abrasivität im Sinne einer mittleren Polierwirkung der Zahnoberfläche ohne Schädigung des Zahnschmelzes und
— verbesserte Lagerstabilität in Hinblick auf eine gleichbleibende Abrasivität und Verdickung (ohne Nachverdickung!) für die Dauer von mindestens einem Jahr.

In einer bestimmten Ausführungsform kann man der Zahnpflegemittel-Rezeptur als Additiv 0,5—5 Gew.-% Polyäthylenglykol mit einem Molekulargewicht im Bereich von 400 bis 2 000 als Verdickungsmittel zufügen. Diese Maßnahme bewirkt entweder bei gleichbleibender Endviskosität des Zahnpflegemittels die Einsparung an Putzkörper oder bei gleichbleibender Putzkörper-Konzentration eine erhöhte Viskosität der Zahnpaste (z.B. unterschiedlich bei der Tuben- oder Spenderabfüllung von Zahnpasten).

Durch die Kombination der im erfindungsgemäßen Zahnpflegemittel verwendeten $SiO_2$-Putzkörper mit verdickend wirkenden Additiven wie Polyäthylenglykol, pyrogener oder gefällter Kieselsäure werden dem Zahnkosmetiker Mittel anhand gegeben, die es ihm ermöglichen, den Anwendungszweck der erfindungsgemäßen Zahnpflegemittel erheblich auszudehnen. Es darf dabei nicht außer Acht gelassen werden, daß auch diese Varianten aus der Sicht der Lagerstabilität des Zahnpflegemittels deutlich verbesserte Eigenschaften aufweisen.

Selbstverständlich lassen sich die erfindungsgemäßen Zahnpflegemittel auch in transparenter und opaker Form herstellen.

Transparente Zahnpasten Hauptbestandteile: Wasser, Glyzerin, Putzkörper. Als Putzkörper Siliciumdioxid (Kieselsäure). Nebenbestandteile, Bindemittel z.B. CMC, Konservierungsmittel (Natriumbenzoat), Süßstoff (Saccharin), Aromaöle, Schaummittel (z.B. Sulfonate), Wirkstoffe z.B. Fluoride, Farbstoffe.

Opake Pasten Hauptbestandteile: Putzkörper, Wasser und Sorbitol. Putzkörper können hier auch Kreiden, Aluminiumhydroxide und Calciumphosphate ausser Kieselsäuren und amorphen Silikaten (Natriumaluminiumsilikate) sein. Nebenbestandteile, Bindemittel (z.B. CMC), Konservierungsmittel (Na-Benzoat), Süßstoff (Saccharin), Opazifierungsmittel ($TiO_2$), Wirkstoffe (Fluoride), Schaumbildende Stoff (Sulfonate), gegebenenfalls Farbstoffe.

Die erfindungsgemäßen Zahnpflegemittel werden anhand der folgenden Beispiele im Vergleich zu Zahnpflegemitteln des Standes der Technik näher erläutert und beschrieben:

Die zur Herstellung der Zahnpflegemittel verwendeten Fällungskieselsäuren, pyrogenen Kieselsäuren und Kieselsäure-Xerogele sind in der Übersichtstabelle Nr. 1 auf der Seite 6 mit ihren für die Anwendung in Zahnpasten relevant erachteten physikalisch-chemischen Daten zusammengefäßt. Im Beispielteil wird in Rahmen der Rezeptur und Ergebnisbeschreibung von den Buchstaben A-I Gebrauch gemacht. Die Buchstaben A-D bezeichnen die in dem erfindungsgemäßen Zahnpflegemittel verwendeten Abrasivkieselsäuren. Neben den tabellarisch aufgeführten Kieselsäure-haltigen Zahnpastenfüllstoffen werden noch folgende Füllstoffe für Vergleichsversuche herangezogen:

Kreide ($CaCO_3$); Hersteller: Schäfer, Diez/Lahn Dicalziumphosphatdihydrat; Hersteller: Benckiser-Knapsack Titandioxid RN 56; Hersteller: Kronos AG

Alle anderen Zahnpasten-Rohstoffe, die in den aufgeführten Rezepturen enthalten sind, werden in der Übersichtstabelle Nr. 2 alphabetisch geordnet aufgelistet und ein Lieferantennachweis erbracht.

Zur Demonstration der technischen Effekte und für Vergleichsuntersuchungen werden im Beispielteil im wesentlichen vier Grundrezepteuren verwendet, und zwar:

— Eine opake Sorbitol-Paste, die 40 Gew.-% Sorbitol als Hauptbestandteil enthält.
— Eine transparente Glyzerin-Paste, die 60 Gew.-% Glyzerin als Hauptbestandteile enthält.
— Eine Kreide-Paste mit Glyzerin.
— Eine Phosphat-Paste mit Sorbitol und Glyzerin.

Die genaue Zusammensetzung der untersuchten Pasten wird jeweils in den entsprechenden Beispielteilen näher angegeben. Dabei wird in der Regel eine Zunahme an Putzkörper durch eine Abnahme an Wasser kompensiert.

Zur Herstellung der Pasten werden die Komponenten in einer RETSCH-Mühle angeteigt, dreimal auf dem Dreiwalzenstuhl homogenisiert und anschließend mittels Vakuumpumpe im Exsiccator entlüftet,

Beschreibung der Prüf- und Meßmethoden:

(Siehe Tabellen Seite 6 ff.)

Fallungskleselsäuren, pyrogene Kieselsäuren und Kieselsäure-Xerogele, deren Anwendung
in Zahnpilegemitteln des Beispielteiles beschrieben ist.

| Physik.-chem. Kenndaten | Abrasiv-Kiesel-säure | Abrasiv-Kiesel-säure | Abrasiv-Kiesel-säure | Abrasiv-Kiesel-säure | Handels-übliches $SiO_2$-Xe-rogel Syloid AL 1 | Handels-übliches $SiO_2$-Xe-rogel Syloid 63 | Handels-übliche Fällungs-Kiesel-säure FK 320DS | Handels-übliche pyrogene Kiesel-säure Aerosil 200 V | Handels-übliche Fällungs-kiesel-säure FK 300 DS |
|---|---|---|---|---|---|---|---|---|---|
| Type: | A | B | C | D | E | F | G | H | I |
| BET-Oberfläche $m^1/g$ | 103 | 101 | 70 | 51 | 633 | 719 | 170 | 200 | 300 |
| Sekundärteilchen-größenverteilung n. Coulter Counter | Fig. 1 | Fig. 2 | Fig. 2 | Fig. 2 | Fig. 3 | Fig. 4 | Fig. 2 | — | Fig. 2 |
| "ALPINE"-Siebrückst. >63 μm (Gew.-%) | 1,4 | 0,1 | 0,1 | 0,1 | <0,1 | <0,1 | — | — | — |
| Cu-Abrieb* (mg) | 14 | 8 | 12 | 17 | 7—8 | 11—12 | <1 mg | <1 mg | <1 mg |
| Viskosität** (mPas) | 36000** | — | 38400** | 33500** | nicht meßbar zu dünn-flüssig | nicht meßbar zu dünn flüssig | 52200*** | — | 54000*** |
| Hellbezugswert A(%) | 94,4 | 96,8 | 95,7 | 94,6 | .. | 94,1 | .. | .. | .. |

\* in 10%iger Glyzerin-Dispersion
\*\* in 30%iger Glyzerin-Wasserdispersion (1:1)
\*\*\* in 15%iger Glyzerin-Wasserdispersion (1:1)

EP 0 063 222 B2

TABELLE Nr. 2

Zahnpasten-Rohstoff und Lieferantennachweis (ausgen. Füllstoffe)

| Rohstoff | Chemische Bezeichnung | Firma |
|---|---|---|
| Aromaöl | Pfefferminzöl Wintergrünöl | Haarmann u. Reimer, Dragoco |
| Dehydazol A 400 P | Carboxymethylcellulose | Henkel |
| Farblösung 0,5%ig | Lebensmittelfarbstoffe | Siegle-Farben |
| Glyzerin | Glyzerin DAB 7 | Merck |
| Polyäthylenglykol 400 | Polyäthylenglykol | Merck |
| Polyäthylenglykol 1500 | Polyäthylenglykol | Merck |
| Paraffinöl | Paraffin | Merck |
| Saccharin | Benzoesäuresulfimid | Bayer |
| Saccharin Natrium | Natriumsalz des Benzoesäuresulfimids | Bayer |
| Solbrol M | p-Oxybenzoesäuremethyl ester | Bayer |
| Solbrol M-Na | Natriumsalze des p-Oxy- benzoesäuremethylester | Bayer |
| Sorbitol | Sorbitol | Maizena |
| | Karion F (70%ige wäss- rige Zuckerlösung) | Merck |
| Texapon K 12 | Nariumlaurylsulfonat | Henkel |

**Bestimmung des Cu-Abrieb in 10%iger glyzerindispersion**

a) Herstellung der Glyzerindispersion
153 g Glyzerin (DAB 7; Dichte = 1,26 g/ml), wasserfrei, werden in einem Polyäthylenbecher (250 ml) eingewogen. Mit dem Spatel werden vorsichtig 17 g Putzkörper untergemischt. Die Mischung wird anschließend mit einem Flügelrührer (Durchmesser 5,2 cm) 12 Minuten lang bei 1 500 Upm homogenisiert.

b) Durchführung der Abriebmessung
Die Bestimmung der Abriebmessung erfolgt in dem Abriebtestgerät, welches gemäß den folgenden Druckschriften bekannt ist: (1) Pfrengle: Fette, Seifen, Anstrichmitte, *63* (5) (1961), Seiten 445 bis 451 "Abrasion und Reinigungskraft von Putzkörpern in Zahnpasten" (2) A. RENG. F. DANY, Parfümerie und Kosmetik *59* (2) (1978), Seiten 37 bis 45; "Anwendungstechnische Prüfung von Zahnpasten". Dazuwerden die 6 Tröge des Testgerätes mit je 20 ml der homogenen Dispersion beschichtet. Der Abrieb, den sechs plangeschliffene Nylonbursten an sechs plangeschliffenen Zu-Blechen (Elektrolyt-Kupfer) in funf Stunden mit 50 000 Doppelhüben bewirken, wird durch Differenzwägung bestimmt. Bei der Berechnung der Abrasivität werden von den erhaltenen Werten Mittelwerte gebildet. Der Abrieb (Abrasivitat) wird in mg Cu angegeben.

Bestimmung des Cu-Abriebs von opaken und transparenten Zahnpasten

a) Herstellung der Zahnpasten

Siehe obige Beschreibung auf Seite -13-b) Durchführung der Abriebmessung

**EP 0 063 222 B2**

### 1. Grundlage

Eine wichtige Aussage über die Qualität einer Zahnpaste ist die Abrasivität, die in der Regel durch die RDA-Methode und durch die Kupferabriebmethode bestimmt wird. Die erhaltenen Werte geben einen wichtigen Aufschluß darüber, wie die Paste in der Reinigungskraft und im Abrieb auf den Zahnschmelz und Zahnhals wirkt. Dazu wird mit der Zahnpaste und mit Glyzerin eine Suspension hergestellt. Von dieser Mischung wird die Abrasivität mit der Abriebmaschine bestimmt.

### 2. Geräte Reagenzien

Dreiwalzenstuhl (Optimat 1)
Abriebmaschine
Analysenwaage, Ofen
Glyzerin DAB 7 (Dichte g/ml = 1,26) wasserfrei
Zahnpasten

### 3. Durchführung

#### 3.1. Herstellung der Pastensuspension

in 100 g der Zahnpaste werden in einer Porzellanschale 75 g Glyzerin mit einem Spatel eingerührt und anschließend 4× auf dem Dreiwalzenstuhl homogenisiert. Von der erhaltenen Pasten-Suspension wird der Abrieb bestimmt.

#### 3.2 Abriebmessung

Die Bestimmung erfolgt in dem Abriebtestgerät. Sechs gereinigte, plangeschliffene, genau gewogene Elektrolyt-Kupferbleche werden in die sechs Träge des Testgerätes eingelegt. Anschließend werden je 20 ml der homogenen Dispersion eingefüllt. Auf die Cu-Bleche werden plangeschliffene Nylonbursten aufgesetzt. Die Durchführung erfolgt mit 50 000 Doppelhüben (ca. 5 Stunden). Nach Beendigung der Bestimmung werden die Kupferbleche zunächst mit Wasser und anschließend mit Aceton gespült. Die Trocknung erfolgt in einem Exsiccator. Die vollkommen trocknen Cu-Bleche werden genau gewogen und die Gewichtsdifferenz in mg Cu als Abrasivität angegeben. Bei der Auswertung werden eventuelle Ausreißer eliminiert und von den restlichen Werten der Mittelwert gebildet.

### 4. Auswertung

Die Abrasivität wird in mg Cu angegeben.
Gewicht Cu-Blech vor der Bestimmung minus Gewicht Cu-Blech nach der Bestimmung
= Verlust an Elektrolykupfer in mg
= Abrasivität

Prüfung der rheologischen Wirksamkeit von Abrasivkieselsäuren in 30%iger Glyzerin/Wasser-Dispersion (Gemisch 1:1)

### 1. Probenansätze

60 g Kieselsäure
70 g Glyzerin wasserfrei DAB 7, Dichte ml/g 1,26
70 g Wasser dest.
───
200 g = 30%ige Dispersion bezogen auf Kieselsäure

### 2. Durchführung

Die Abrasivkieselsäuren wurden von Hand in einem 400 ml-Becherglas (breite Form) in das Glyzerin/Wasser-Gemisch mit einem Glasstab eingerührt (1 Min.) und 24 Stunden stehen gelassen.

Danach wird die Wiskosität gemessen.

### 3. Messung

Die Viskositätsmessung wird im selben Becherglas mit dem Brookfield-Viskosimeter RVT Spindel 5 bei verschiedenen Upm. durchgeführt.

### 4. Ausrechnung

Abgelesener Skalenwert × Faktor = Viskosität in mPas.

Prüfung der rheologischen Wirksamkeit von Verdickungskieselsäuren in 15%iger Glyzerin/Wasser-Dispersion (Gemisch 1:1)

8

1. Probenansatz

    30 g Kieselsäure
    85 g Glyzerin wasserfrei DAB 7, Dichte g/ml 1,26
    85 g H$_2$O dest.
    ———

    200 g 15%ige Dispersion bezogen auf Kieselsäure

2. Durchführung

Die Verdickungskieselsäure wird von Hand in ein 400 ml-Becherglas (breite Form) in das Glyzerin/ Wasser-Gemisch mit einem Glasstab eingerührt (ca. 1 Min.) und 24 Std. stehen gelassen. Danach wird die Viskosität gemessen.

3. Messung

Die Viskositätsmessung wird im selben Becherglas mit dem Brookfield-Viskosimeter RVT Spindel 5 bei verschiedenen Upm. durchgeführt.

4. Ausrechnung

Abgelesener Skalenwert × Faktor = Viskosität in mPas

Bestimmung der Viskosität von Zahnpasten:
    a) Herstellung der Zahnpasten
    Siehe obige Beschreibung auf Seite -13-

    b) Durchführung der Viskositätsmessung

Prüfung der Zahnpastenviskosität mit dem Rotoviskosimeter: System Platte-Kegel.

1. Gerät

Rotovisko der Firma Haake Karlsruhe, mit Thermostat Meßkopf 50 Platte-Kegel Nr. 8012 und 1224.

2. Ausführung

Die Zahnpaste wird zunächst auf 20—22°C eingestellt und dann eine Spatelspitze auf die Platte aufgebracht. Die Messung erfolgt bei Geschwindigkeitsstufe 27. Nach 30 Sekunden wird vom Anzeigeninstrument der Skalenwert abgelesen. Nach dieser Zeit ist ein annähernd konstanter Wert erreicht.

3. Auswertung

Aus Geschwindigkeit, Zeigerwert und Eichkonstante des verwendeten PK Systems erhält man die Viskosität der Paste nach der Gleichung

$$V = K \cdot S \cdot U \ [\text{mPas}]$$

V = Viskosität in mPas
U = Geschwindigkeitsstufe des Rotovisko
s = angezeigter Skalenwert
K = Eichkonstante des verwendeten Kegels

Bestimmung des pH-Wertes von Zahnpasten
a) Herstellung der Zahnpasten

Siehe obige Beschreibung auf Seite -13-

b) Durchführung der pH-Wertmessung

Der pH-Wert von Zahnpasten ist äußerst wichtig, kann doch von dieser Meßgröße die Stabilität einer Paste stark abhängig sein. Extrem alkalische und saure Pasten sind in der Regel unerwünscht, da dadurch Bombage und Korrosion der Al-Tuben zu befürchten ist. Zudem wird in medizinischen Pasten der Wirkstoff chemisch angegriffen und zersetzt. Pasten mit neutralem pH-Wert werden deshalb bevorzugt.

1. Gerät
Labor pH-Meter Type 26 Firma Knick mit Glaselektrode.

2. Durchführung
Die Glaselektrode wird in die Zahnpasta eingetracht und der pH-Wert nach ca. 3 Min. direkt auf dem Anzeigegerät abgelesen.

Visuelle Beurteilungsmethoden von Zahnpflegemitteln
a) Beuerteilung der Strangstabilität einer Zahnpaste

1. Ausführung
Die Zahnpaste wird in eine AL-Tube abgefüllt und anschließend ein ca. 5 cm langer Strang auf eine Glasplatte ausgedrückt.

2. Beurteilung
Die Stabilität des Stanges wird nach 5 Min. beurteilt. Ist die Konsistenz der Paste ungenügend, läuft der Strange optisch sichtbar auseinander. Je nach Breite des zu beurteilenden Stranges wird die Note 1—4 vergeben.
b) Beurteilung der Transparenz einer transparenten Zahnpaste

1. Ausführung
a) die Paste wird zwischen 2 Glasplatten ausgedrückt
b) die Plaste wird auf ein weißes Blatt mit schwarzen Linien gegeben.

2. Beurteilung
zu a) Die Transparenz der Paste ist sehr gut zu beurteilen, da eine nicht genügende Qualität sofort durch eine Trübung sichtbar wird. Je nach Stärke der Trübung wird die Note 1—4 vergeben.
zu b) Bei einer guten Transparenz ist die schwarze Linie durch den Pastenstrang sehr gut zu erkennen. Bei einer schlechteren Transparenz verschwimmt die Linie mehr oder weniger stark. Noten von 1—4.
c) Beurteilung der Separation von Zahnpasten

1. Ausführung
Die Paste wird in AL-Tuben 1/4 Jahr bei 45°C gelagert und anschließend ein ca. 5 cm langer Strang auf eine Loschblattunterlage gedrückt.

2. Beurteilung
Ist eine Separationsneigung vorhanden, ist optisch gut sichtbar vom Strang weg ein Feuchtigkeitsfilm im Löschblatt zu sehen. Je nach Breite dieses Films wird eine Beuteilung von 1—4 gegeben.

Bemerkung
Die aufgezeigte Methode ist in der Regel zu verwenden, da in wenigen Fällen die Separation so stark ausgeprägt ist, daß bereits am Tubenausgang eine Flüssigkeitsschicht gebildet wird.

### Beispielreihe 1

Mit Hilf dieser Beispielreihe soll nachgewiesen werden, daß das im Patentanspruch 1 beschriebene, erfindungsgemäße Zahnpflegemittel mit dem darin spezifizierten Putzkörper mit der Doppelfunktion Verdickung und Abrasivität ohne Additive im Hinblick auf Abrasivität und Konsistenz den heutigen Marktanforderungen genügt, wenn man von opaken Zahnpasten ausgeht. Handelsübliche Putzkörper auf Kieselsäuregelbasis zeigen diese Eigenschaften nicht, was durch vergleichende Untersuchungen der Beispielreihe 1 dargelegt wird.
Die Zusammensetzung der untersuchten opaken Pasten ist in Tabelle Nr. 3 näher beschrieben (Rezepturen 1—12).

Tabelle Nr. 4 enthält die Ergebnisse (pH-Wert, Cu-Abrieb, Viskosität, Strangstabilität und Separation) der an den Rezüpturen 1—12 vorgenommenen Messungen. Daraus folgt:

— Die erfindungsgemäßen Rezepturen Nr. 3 und Nr. 6 bilden mit jeweils 25 Gew.% der Putzkörper B oder C strangstabile Zahnpasten, deren Abriebwerte im Bereich von 8—13 mg Cu voll den Marktanforderungen genügen.

— Die erfindungsgemäße Rezeptur Nr. 9 bildet mit jeweils 25 Gew.-% des härteren Putzkörpers D eine Paste, die ohne Additive noch zum Fließen neigt. Ihre Abrasivwerte erfüllen jedoch mit 16—18 mg Cu die Anforderungen, die im allgemeinen an gut reinigende Zahnpasten, wie z.B. Raucherpasten gestellt werden.

— Die mit 25 Gew.-% an handelsüblichen Putzkörpern formulierte Rezepteur Nr. 12 bildet keine strangstabile Zahnpaste. Ferner liegt selbst bei derartige hohen Füllungsgraden die Abrasivwirkung mit 4—6 mg Cu-Abrieb sehr niedrig.

(Siehe Tabellen Seite 12 ff.)

## TABELLE Nr. 3

### Opake Zahnpflegemittel: Doppelfunkion von Putzkörpern. Rezepturen ohne Additive

| Rezeptur | Lfde Nr. 1 | 2 | 3 | 4 | 5 | 6 | 7 | 8 | 9 | 10 | 11 | 12 |
|---|---|---|---|---|---|---|---|---|---|---|---|---|
| Wasser dest. | 40,35 | 35,35 | 30,35 | 40,35 | 35,35 | 30,35 | 40,35 | 35,35 | 30,35 | 40,35 | 35,35 | 30,35 |
| Dehydazol A 400 P | 1,00 | →| | | | | | | | | | |
| Solbrol M-Ma | 0,15 | →| | | | | | | | | | |
| Saccharin-Na | 0,10 | →| | | | | | | | | | |
| Sorbitol | 40,00 | →| | | | | | | | | | |
| TiO RN56 | 0,40 | →| | | | | | | | | | |
| Paraffinöl | 0,50 | →| | | | | | | | | | |
| Aromaöl | 0,50 | →| | | | | | | | | | |
| Texapon K 12 | 2,00 | →| | | | | | | | | | |
| Kieselsäure B | 15,00 | 20,00 | 25,00 | — | — | — | — | — | — | — | — | — |
| Kieselsäure C | — | — | — | 15,00 | 20,00 | 25,00 | — | — | — | — | — | — |
| Kieselsäure D | — | — | — | — | — | — | 15,00 | 20,00 | 25,00 | — | — | — |
| Kieselsäure E | — | — | — | — | — | — | — | — | — | 15,00 | 20,00 | 25,00 |
| Summe: | 100,00 | →| | | | | | | | | | |

EP 0 063 222 B2

TABELLE Nr. 4

Opake Zahnpflegemittel: Doppelfunktion von Putzkörpern Ergebnisse ohne Additive

| Rezeptur/Eigenschaften Lfd. Nr. | pH-Wert | Abrieb mg Cu | Viskosität mPas | Stangestabilität | Separation |
|---|---|---|---|---|---|
| 1 | 5,0 | 7—8 | 1116 | Note 4 | Note 1 |
| 4 | 5,2 | 8—9 | 930 | Note 4 | Note 1 |
| 7 | 4,8 | 12—15 | 806 | Note 4 | Note 1 |
| 10 | 4,1 | 2—3 | zu dünn | Note 4 | Note 3 |
| 2 | 5,3 | 7—9 | 1860 | Note 3 | Note 1 |
| 5 | 6,2 | 8—11 | 1736 | Note 3 | Note 1 |
| 8 | 5,8 | 13—16 | 1364 | Note 4 | Note 1 |
| 11 | 4,2 | 2—3 | 868 | Note 4 | Note 3 |
| 3 | 5,4 | 8—10 | 3348 | Note 1 | Note 1 |
| 6 | 5,8 | 11—13 | 2914 | Note 1 | Note 1 |
| 9 | 5,4 | 16—18 | 2604 | Note 3 | Note 1 |
| 12 | 4,0 | 4—6 | 1860 | Note 3 | Note 1 |

Beispielreihe 2

Mittels dieser Beispielreihe soll gezeigt werden, daß das im Patentanspruch 2 beschriebene erfindungsgemäße Zahnpflegemittel mit dem darin näher spezifizierten Putzkörper mit der ausgeprägten Doppelfunktion Verdickung und Abrasivität mit Additiven auf der Basis von Polyäthylenglykolen im Hinblick auf Abrasivität, Transparenz und Konsistenz den gegenwärtigen Marktanforderungen genügen, wenn man von transparenten Zahnpasten ausgeht. Handelsübliche Putzkörper auf Kieselsäuregelbasis zeigen diese Eigenschaften nicht, was durch vergleichende Tests der Beispielreihe 2 dargelegt wird.

Die Zusammensetzung der untersuchten transparenten Pasten ist in Tabelle Nr. 5 näher beschrieben (Rezepturen 1—12).

(Siehe Tabellen Seite 15 ff.)

TABELLE Nr. 5

Transparente Zahnpflegemittel: Doppelfunktion von Putzkorpern, Rezepturen mit Additiv

| Rezeptur | Lfd Nr. 1 | 2 | 3 | 4 | 5 | 6 | 7 | 8 | 9 | 10 | 11 | 12 |
|---|---|---|---|---|---|---|---|---|---|---|---|---|
| Wasser dest. | 17,95 | 17,95 | 12,95 | 12,95 | 17,95 | 17,95 | 12,95 | 12,95 | 17,95 | 17,95 | 12,95 | 12,95 |
| Farblsg. blau | 0,50 | | | | | ⟶ | | | | | | |
| Dehydazol A 400 P | 0,50 | | | | | ⟶ | | | | | | |
| Solbrol M-Na | 0,15 | | | | | ⟶ | | | | | | |
| Saccharin-Na | 0,10 | | | | | ⟶ | | | | | | |
| Glyzerin | 60,00 | | | | | ⟶ | | | | | | |
| Polyäthylen-glykol 400 | 3,50 | — | 3,50 | — | 3,50 | — | 3,50 | — | 3,50 | — | 3,50 | — |
| Polyäthylen-glykol 1.500 | — | 3,50 | — | 3,50 | — | 3,50 | — | 3,50 | — | 3,50 | — | 3,50 |
| Aromaöl | 1,00 | | | | | ⟶ | | | | | | |
| Texapon K 12 | 1,30 | | | | | ⟶ | | | | | | |
| Kieselsäure A | 15,00 | 15,00 | 20,00 | 20,00 | — | — | — | — | — | — | — | — |
| Kieselsäure C | — | — | — | — | 15,00 | 15,00 | 20,00 | 20,00 | — | — | — | — |
| Kieselsäure E | — | — | — | — | — | — | — | — | 15,00 | 15,00 | 20,00 | 20,00 |
| Summe: | 100,00 | | | | | ⟶ | | | | | | |

# EP 0 063 222 B2

## TABELLE Nr. 6

### Transparente Zahnpflegemittel: Doppelfunktion von Putzkörpern mit Additiv Ergebnisse

| Rezeptur Lfd. Nr. | pH-Wert | Abrieb mg Cu | Viskosität mPas | Strangstabilität | Separation | Transparenz |
|---|---|---|---|---|---|---|
| 1 | 7.6 | 16 | 1 100 | Note 4 | Note 1 | Note 2 |
| 5 | 7,8 | 14 | 1 302 | Note 4 | Note 1 | Note 2 |
| 9 | 5,9 | 7 | nicht meßbar | Note 4 | Note 4 | Note 4 |
| 2 | 7,2 | 15 | 1 320 | Note 3 | Note 1 | Note 2 |
| 6 | 7,9 | 12 | 1 612 | Note 3 | Note 1 | Note 2 |
| 10 | 6,0 | 5 | nicht meßbar | Note 4 | Note 4 | Note 4 |
| 3 | 7,5 | 18 | 1 980 | Note 3 | Note 1 | Note 2 |
| 7 | 7,7 | 14 | 2.480 | Note 2 | Note 1 | Note 2 |
| 11 | 5,8 | 6 | 868 | Note 4 | Note 1 | Note 4 |
| 4 | 7,4 | 17 | 2 980 | Note 1 | Note 1 | Note 2 |
| 8 | 7,7 | 13 | 3 410 | Note 1 | Note 1 | Note 2 |
| 12 | 5,8 | 6 | 1 550 | Note 3 | Note 1 | Note 4 |

Tabelle Nr. 6 enthält die Ergebnisse (pH-Wert, Cu-Abrieb, Viskosität, Strangstabilität, Separation und Transparenz) der an den Rezepturen 1—12 vorgenommenen Messungen. Daraus folgt:

— Die erfindungsgemäßen Rezepturen Nr. 4 und Nr. 8 bilden mit jeweils 20 Gew.-% der Putzkörper A oder C und 3.5% Polyäthylenglykol 1 500 strangstabile Zahnpasten, deren Abriebwerte von den Marktanforderungen genügen.

— Die mit 20 Gew.-% an handelsüblichen Putzkörper formulierten Rezepturen Nr. 11 und Nr. 12 bilden weder mit 3,5% Polyäthylenglykol 400 noch mit Polyäthylenglykol 1 500 strangstabile Zahnpasten. Die Transparenz die Pasten ist unbefriedigend.

### Beispielreihe 3

Dise Beispielreihe soll beweisen, daß das im Patentanspruch 2 beschriebene erfindungsgemäße Zahnpflegemittel mit den darin näher spezifizierten Putzkörpern mit der Doppelfunktion Verdickung und Abrasivität mit Additiven auf der Basis von Polyäthylenglykol im Hinblick auf Abrasivität und Konsistenz den gegenwärtigen Marktanforderungen genügt und im Hinblick auf die einzusetzende Menge an Putzkörper sogar übertrifft.

Handelsübliche Putzkörper auf Kieselsäuregelbasis zeigen diese Eigenschaft selbst bei Verwendung von 5% Polyäthylenglykol 1 500 nicht, was durch vergleichende Prüfungen der Beispielreihe 3 dargelegt wird.

Die Zusammensetung der untersuchten opaken Pasten ist in Tabelle Nr. 7 näher beschrieben (Rezepturen 1—12).

Tabelle Nr. 8 enthält die Ergebnisse (pH-Wert, Cu-Abrieb, Viskosität, Strangstabilität und Separation) der an der Rezepturen 1—12 vorgenommenen Messungen. Daraus folgt:

— Die erfindungsgemäßen Rezepturen Nr. 4, Nr. 7 und Nr. 8 bilden mit jeweils 20 Gew.-% der Putzkörper A oder C und 2 Gew.-% bzw. 5 Gew.-% Polyäthylenglykol 1 500 strangstabile Zahnpasten, deren Abriebwerte voll den Marktanforderungen genügen.

— Die mit 20 Gew.-% an handelsüblichen Putzkörpern formulierten Rezepturen Nr. 9, Nr. 10, Nr. 11 und Nr. 12 bilden weder mit 2 Gew.-% noch mit 5 Gew.-% Polyäthylenglykol 400 oder Polyäthylenglykol 1 500 strangstabile Zahnpasten.

(Siehe Tabellen Seite 18 ff.)

Beispielreihe 4

Die folgende Beispielreihe soll die deutliche Überlegenheit der in den erfindungsgemäßen Zahnpflegemitteln verwendeten Putzkörper im Hinblock auf ihre Abrasion als auch ihre Verdickungswirkung im Vergleich zu den handelsüblichen Putzkörpern auf der Basis von Kieselsäuregelen demonstrieren. Dabei wird zur besseren Hervorhebung der Unterschiede auf die Verwendung von verdickend wirkender Fällungskieselsäure — z.B. FK 320 DS — zurückgegriffen. Die Prüfungsergebnisse zeigen, daß man durch die Wahl des Verhältnisses von Abrasiv- zu Verdickungskomponente bei konstantem Füllungsgrad von 22 Gew.-% die Abrieb- und Verdickungwirkung in den erfindungsgemäßen Zahnpflegemitteln variieren kann. Im Vergleich dazu ist bei der Verwendung handelsüblicher Putzkörper dieser Spielraum sehr stark eingeschränkt.

Die Zusammensetzung der untersuchten, opaken Zahnpasten ist in Tabelle Nr. 9 näher beschrieben (Rezepturen 1—20).

Tabelle Nr. 10 enthält die Ergebnisse (pH-Wert, Cu-Abrieb und Viskosität) der an den Rezepturen 1—20 vorgenommenen Messungen. Daraus folgt für die Rezepturen 1—10:

— Die erfindungsgemäßen Rezepturen Nr. 1—Nr. 5 mit einem Gesamt-Kieselsäuregehalt von 22 Gew.-% genügen ausnahmslos den Anforderungen eines marktgerechten Zahnpflegemittels. Dies gilt auch für solche Formulierungen, die keine oder nur 2 Gew.-% einer verdickend wirkenden Fällungskieselsäure (Type G) enthalten. Bei Steigerung des Gehaltes an Verdickungskomponente bei gleichzeitiger Absenkung der Putzkörperkomponente (Typ C) werden sehr strangstabile Zahnpasten erhalten, die aufgrund ihres relativ hohen Viskositätsniveau Einsparungen an Verdickungskieselsäure gestatten. Im Gegensatz dazu sind die im Vergleich geprüften Rezepturen Nr. 8—Nr. 10 mit gleichem Gesamtkieslesäuregehalt mit dem Putzkörper Typ E noch zu dünnflüssig. Erst bei den Rezepturen Nr. 6 und Nr. 7, die Gehalt von 12 und 14 Gew.-% an Verdickungskieselsäure (Typ G) enthalten, werden Zahnpasten mit brauchbarer Konsistenz erhalten.

— Bei den erfindungsgemäßen Rezepturen Nr. 1—Nr. 5 als auch bei den Vergleichs-Rezepturen Nr. 6—Nr. 10 erhöht sich mit steigendem Anteil an Putzkörper Typ C oder Typ E die Abrasivität nicht linear. Die Steigerung an Abrasivität ist jedoch mit den in den erfindungsgemäßen Rezepturen mit Putzkörper vom Typ C absolut gesehen ausgeprägter als bei den Vergleichsrezepturen mit handelsüblichen Putzkörper vom Typ E.

TABELLE Nr. 7

Opake Zahnpflegemittel: Doppelfunktion von Putzkörpern, Rezepturen mit Additiv

| Rezeptur | Lfd Nr. 1 | 2 | 3 | 4 | 5 | 6 | 7 | 8 | 9 | 10 | 11 | 12 |
|---|---|---|---|---|---|---|---|---|---|---|---|---|
| Wasser dest. | 33,35 | 30,35 | 33,35 | 30,35 | 33,35 | 30,35 | 33,35 | 30,35 | 33,35 | 30,35 | 33,35 | 30,35 |
| Dehydrazol A400P | 1,00 | ──────────────────────────────────────→ | | | | | | | | | | |
| Solbrol M-Na | 0,15 | ──────────────────────────────────────→ | | | | | | | | | | |
| Saccharin-Na | 0,10 | ──────────────────────────────────────→ | | | | | | | | | | |
| Sorbitol | 40,00 | ──────────────────────────────────────→ | | | | | | | | | | |
| Polyäthylenglykol 400 | 2,00 | 5,00 | — | — | 2,00 | 5,00 | — | — | 2,00 | 5,00 | — | — |
| Polyäthylenglykol 1500 | — | — | 2,00 | 5,00 | — | — | 2,00 | 5,00 | — | — | 2,00 | 5,00 |
| TiO₂ RN 56 | 0,40 | ──────────────────────────────────────→ | | | | | | | | | | |
| Paraffinöl | 0,50 | ──────────────────────────────────────→ | | | | | | | | | | |
| Aromaöl | 0,50 | ──────────────────────────────────────→ | | | | | | | | | | |
| Texapon K12 | 2,00 | ──────────────────────────────────────→ | | | | | | | | | | |
| Kieselsäure A | 20,00 | 20,00 | 20,00 | 20,00 | — | — | — | — | — | — | — | — |
| Kieselsäure C | — | — | — | — | 20,00 | 20,00 | 20,00 | 20,00 | — | — | — | — |
| Kieselsäure E | — | — | — | — | — | — | — | — | 20,00 | 20,00 | 20,00 | 20,00 |
| Summe: | 100,00 | ──────────────────────────────────────→ | | | | | | | | | | |

EP 0 063 222 B2

TABELLE Nr. 8

Opake Zahnpflegemittel: Doppelfunktion von Putzkörpern mit Additiv Ergebnisse

| Reseptur Lfd. Nr. | pH-Wert | Abrieb mg Cu | Viskosität mPas | Strangstabilität | Separation |
|---|---|---|---|---|---|
| 1 | 5,6 | 13 | 1 860 | Note 3 | Note 1 |
| 5 | 5,9 | 12 | 2 348 | Note 2 | Note 1 |
| 9 | 4,0 | 5 | 1 040 | Note 4 | Note 1 |
| 2 | 5,7 | 12 | 2 120 | Note 2 | Note 1 |
| 6 | 5,5 | 10 | 2 506 | Note 2 | Note 1 |
| 10 | 4,1 | 4 | 1 320 | Note 3 | Note 1 |
| 3 | 5,5 | 15 | 2 450 | Note 2 | Note 1 |
| 7 | 5,8 | 12 | 2 640 | Note 1 | Note 1 |
| 11 | 4,0 | 6 | 1 220 | Note 3 | Note 1 |
| 4 | 5,6 | 14 | 3 120 | Note 1 | Note 1 |
| 8 | 5,9 | 13 | 3 320 | Note 1 | Note 1 |
| 12 | 4,1 | 6 | 1 580 | Note 3 | Note 1 |

Für die in der Tabelle Nr. 10 enthaltenen Ergebnisse der an der Rezepturen Nr. 11—Nr. 20 gemessenen Werte gilt folgende Aussage:

— Die erfindungsgemäßen Rezepturen Nr. 11—Nr. 20 mit einem Gesamtkieselsäuregehalt von 22 Gew.-% genügen weitgehend den Anforderungen eines markgerechten zahnpflegemittels. Hier tendieren die Formulerungen Nr. 14, Nr. 15, Nr. 19 und Nr. 20, die keine oder nur 2 Gew.-% einer verdickend wirkenden Fällungskieselsäure (Type G) enthalten, zum Fließen. Bei Steigerung des Gehaltes an Verdickungskomponentente bei gleichzeitiger Absenkung der Putzkörper-Komponente (Typen A und D) werden sehr strangstabile Zahnpasten erhalten, deren Rezepturen aufgrund ihres relativ hohen Verdickungsniveaus Einsparungen an Verdickungskieselsäure gestatten.

— Bei den erfindungsgemäßen Putzkörpern vom Typ A und Typ D lassen sich je nach Zusammensetzung der Rezepturen aus Abrasiv- und Verdickungskomponente Cu-Abriebwerte im Bereich von 11—18 mg Cu maßgeschneidert einstellen. Dabei bleibt selbst für den Grenzfall der Anwendung von nur Abrasiv-Komponente die Strangstabilität gut. Es zeigt sich jedoch, daß die Cu-Abriebwerte in keiner Weise linear mit der Konzentration der Abrasiv-Komponente ansteigen, andererseits jedoch die Abrasivität der erfindungsgemäßen Zahnpflegemittel in relativ breiten Grenzen wählbar ist, ohne auf andere wichtige Eigenschaften des Zahnpflegemittels wie Strangstabilität und Separationsstabilität verzichten zu müssen. Dies gilt nicht für den Putzkörper des handelsüblichen Putzkörpers (Typ E) auf Kieselgelbasis.

(Siehe Tabellen Seite 21 ff.)

## TABELLE Nr. 9

Opake Zahnpflegemittel: Doppelfunktion von Putzkörpern. Rezepturen mit verdickend wirkenden Fällungskieselsäuren

| Rezeptur | Lfd. Nr.<br>1 | 2 | 3 | 4 | 5 | 6 | 7 | 8 | 9 | 10 |
|---|---|---|---|---|---|---|---|---|---|---|
| Wasser dest. | 33,40 | → | | | | | | | | |
| Dehydrazol A 400 P | 1,00 | → | | | | | | | | |
| Solbrol M | 0,15 | → | | | | | | | | |
| Saccharin-Na | 0,05 | → | | | | | | | | |
| Sorbitol | 30,00 | → | | | | | | | | |
| Glyzerin | 10,00 | → | | | | | | | | |
| TiO$_2$ RN 56 | 0,40 | → | | | | | | | | |
| Paraffinöl | 0,50 | → | | | | | | | | |
| Aromaöl | 0,50 | → | | | | | | | | |
| Texapon K 12 | 2,00 | → | | | | | | | | |
| Kieselsäure C | 8,00 | 10,00 | 14,00 | 20,00 | 22,00 | — | — | — | — | — |
| Kieselsäure E | — | — | — | — | — | 8,00 | 10,00 | 14,00 | 20,00 | 22,00 |
| Kieselsäure G | 14,00 | 12,00 | 8,00 | 2,00 | — | 14,00 | 12,00 | 8,00 | 2,00 | — |
| Summe: | 100,00 | → | | | | | | | | |

EP 0 063 222 B2

### TABELLE Nr. 9 (Forsetzung)
Opake Zahnpflegemittel: Doppelfunktion von Putzkörpern. Rezepturen mit verdickend wirkenden Fällungskieselsäuren

| Rezeptur | Lfd. Nr. 11 | 12 | 13 | 14 | 15 | 16 | 17 | 18 | 19 | 20 |
|---|---|---|---|---|---|---|---|---|---|---|
| Wasser dest. | 33,40 | | | | | | | | | → |
| Dehydrazol A 400 P | 1,00 | | | | | | | | | → |
| Solbrol M | 0,15 | | | | | | | | | → |
| Saccharin-Na | 0,05 | | | | | | | | | → |
| Sorbitol | 30,00 | | | | | | | | | → |
| Glyzerin | 10,00 | | | | | | | | | → |
| TiO$_2$ RN 56 | 0,40 | | | | | | | | | → |
| Paraffinöl | 0,50 | | | | | | | | | → |
| Aromaöl | 0,50 | | | | | | | | | → |
| Texapon K 12 | 2,00 | | | | | | | | | → |
| Kieselsäure A | 8,00 | 10,00 | 14,00 | 20,00 | 22,00 | — | — | — | — | — |
| Kieselsäure D | — | — | — | — | — | 8,00 | 10,00 | 14,00 | 20,00 | 22,00 |
| Kieselsäure G | 14,00 | 12,00 | 8,00 | 2,00 | — | 14,00 | 12,00 | 8,00 | 2,00 | — |
| Summe: | 100,00 | | | | | | | | | → |

EP 0 063 222 B2

TABELLE Nr. 10

Opake Zahnpflegemittel: Doppelfunktion von Putzkörpern. Rezepturen mit verdickend
wirkenden Fällungskieselsäuren. Ergebnisse

| Rezeptur Lfd. Nr. | pH-Wert | Abrieb mg Cu | Viskosität mPas | Beurteilung | Strangstabilität |
|---|---|---|---|---|---|
| 1 | 4,9 | 8—9 | 6.000 | Note 1 | |
| 2 | 5,2 | 9—11 | 5.270 | Note 1 | |
| 3 | 5,3 | 10—12 | 4.650 | Note 1 | |
| 4 | 5,5 | 13—14 | 3.800 | Note 1 | |
| 5 | 5,4 | 13—15 | 3.100 | Note 1 | |
| 6 | 4,4 | 2—3 | 5.200 | Note 1 | |
| 7 | 4,2 | 2—3 | 3.400 | Note 1 | |
| 8 | 4,2 | 3—4 | 2.700 | Note 2 | |
| 9 | 4,1 | 5 | 1.800 | Note 3 | |
| 10 | 4,2 | 5—6 | 1.300 | Note 4 | |

TABELLE Nr. 10 (Fortsetzung)

Opake Zahnpflegemittel: Doppelfunktion von Putzkörpern. Rezepturen mit verdickend
wirkenden Fällungskieselsäuren. Ergebnisse

| Rezeptur Lfd. Nr. | pH-Wert | Abrieb mg Cu | Viskosität mPas | Strangstabilität |
|---|---|---|---|---|
| 11 | 5,0 | 11—13 | 5.952 | Note 1 |
| 12 | 5,2 | 10—12 | 5.230 | Note 1 |
| 13 | 5,5 | 12—14 | 4.430 | Note 1 |
| 14 | 5,0 | 13—15 | 3.100 | Note 2 |
| 15 | 5,5 | 16—18 | 2.840 | Note 2 |
| 16 | 5,0 | 12—14 | 5.890 | Note 1 |
| 17 | 5,0 | 14—16 | 4.960 | Note 1 |
| 18 | 5,1 | 15—17 | 4.340 | Note 1 |
| 19 | 5,4 | 16—18 | 2.914 | Note 2 |
| 20 | 5,0 | 16—18 | 2.790 | Note 2 |

EP 0 063 222 B2

**Patentansprüche**

1. Zahnpflegemittel mit verbesserter Lagerstabilität im Hiblick auf Rheologie und Abrasivität, das dadurch gekennzeichnet, daß es als Verdickungs- und Abrasivmittel eine gefällte Kieselsäure in einer Menge von 20 bis 25 gew.-% enthält, die durch folgende physikalische-chemischen Kenndaten charakterisiert wird:

| | |
|---|---|
| BET-Oberfläche nach DIN 66 131 | $m^2/g$ 15—110 |
| Stampfgewicht | g/l 90—650 |
| Sekundärteilchengrößen-Verteilungkurve gem Fig 2 nach Coulter Counter "ALPINE"-Siebrückstand >63 µm | Gew.-% <0,1 |
| Cu-Abrieb in 10%iger Glyzerin-dispersion | mg 5—30 |
| Viskosität in 30%iger Glyzerin Wasser-Dispersion (1:1) Brookfield RTV Sp 5 | mPas 30.000—60.000 |
| Hellbezugswert A nach DIN 55 921 | % 90—96 |

und dadurch erhältlich ist, daß man eine Originalfällungskieselsäuresuspension, die durch Fällung der Kieselsäure aus einer vorgelegten Alkalisilikatlösung mit einer Konzentration von etwa 5—25 g $SiO_2$ je Liter Lösung mit einer Säure- und Alkalisilikatlösung mit bestimmten Lösungskonzentrationen und bestimmten Zulaufgeschwindigkeiten unter Aufrechterhaltung einer Fälltemperatur, im Reaktionsmedium zwischen 80 und 90°C derart hergestellt wird, daß die Viskosität des Reaktionsmediums in einem Zeitraum von mindestens 30% der gesamten Fälldauer gleichmäßig niedrig und der pH-Wert zwischen 10 und 12 gehalten wird, und die Zugabe der Reaktionsteilnehmer erst dann beendet wird, wenn die Viskosität nach Durchlaufen eines Maximums auf einen weniger als 100% über der Anfangsviskosität liegenden Wert gesunken ist, mit heißem Wasser auf einen Fällungskieselsäuregehalt von 10—30 g/l und Natriumsulfatgehalt von 6—20 g $Na_2SO_4$/l verdünnt, auf 85—95°C erwärmt, den pH-Wert mit Schwefelsäure auf 7 bis 9 einstellt und unter Konstanthalten dieses pH-Wertes durch gleichzeitigen Zulauf von Alkalisilikatlösung. Schwefelsäure und heißem Wasser über eine Fälldauer von 15 bis 180 Minuten eine Fällungskieseläsureendkonzentration von 40—80 g/l einstellt, die Suspension mit konzentrierter Schwefelsäure auf einen pH-Wert unter 7 ansäuert, die Fällungskieselsäure mit Hilfe einer Filterpresse aus der Suspension abstrennt, auswäscht, trocknet und mittels einer Strahlmühle vermahlt.

2. Zahnpflegemittel mit verbesserter Lagerstabilität in Hinblick auf Rheologie und Abrasivität, das dadurch gekennzeichnet ist, daß es als Abrasivmittel in einer Menge von 20 bis 25 Gew.-% eine gefällte Kieselsäure enthält, die durch folgende physikalisch-chemische Kenndaten charakterisiert wird:

| | |
|---|---|
| BET-Oberfläche nach DIN 66 131 | $m^2/g$ 15—110 |
| Stampfgewicht | g/l 150—750 |
| Sekundärteilchengrößenverteilungkurve nach Coulter Counter gemäß Figur 1 "ALPINE"-Siebrückstand >63 µm | Gew.-% <1,5 |
| Cu-Abrieb in 10%iger Glyzerindispersion | mg 5—30 |
| Viskosität in 30%iger Wasser-Glyzerindispersion (1:1) Brookfield RTV Sp 5 | mPas 30 000—60,000 |
| Hellbezugswert A nach DIN 55 921 | % 86—96 |

und dadurch erhätltlich ist, daß man eine Originalfällungskieselsäuresuspension, die durch Fällung der Kieselsäure aus einer vorgelegten Alkalisilikatlösung mit einer Konzentration von etwa 5—25 g $SiO_2$ je Liter Lösung mit einer Säure und Alkalimetallsilikatlösung mit bestimmten Lösungskonzentrationen und bestimmten Zulaufgeschwindigkeiten unter Aufrechterhaltung einer Fälltemperatur im Reaktionsmedium zwischen 80 und 90°C derart hergestellt wird, daß die Viskosität des Reaktionsmediums in einem Zeitraum von mindestens 30% der gesamten Fälldauer gleichmaßig niedrig und der pH-Wert zwischen 10 und 12 gehalten wird, und die Zugabe der Reaktionsteilnehmer erst dann beendet wird, wenn die Viskosität nach Durchlaufen eines Maximums auf einem weniger als 100% über der Anfangsviskosität liegenden Wert gesunken ist, mit heißem Wasser auf einen Fällungskieselsäuregehalt von 10—30 g/l und Natriumsulfatgehalt von 6—20 g $Na_2SO_4$/l verdünnt, auf 85—95°C erwärmt, den pH-Wert mit Schwefelsäure auf 7 bis 9 einstellt und unter Konstanthalten dieses pH-Wertes durch gleichzeitigen Zulauf von Alkalimetallsilikatlösung, Schwefelsäure und gegebenenfalls heißem Wasser über eine Fälldauer von 15 bis 180 Minuten eine Fällungskieselsäureendkonzentration von 40 bis 80 g/l einstellt, die Suspension mit konzentrierter Schwefelsäure auf einen pH-Wert unter 7 ansäuert, die Fällungskieselsäure mit Hilfe einer Filterpresse aus der Suspension abtrennt, auswäscht, trocknet und mittels einer Pendelmühle vermahlt.

3. Zahnpflegemittel nach Ansprüchen 1 bis 2, dadurch gekennzeichnet, daß es zusätzlich 0,5—5 Gew.-%, vorzugsweise 2 bis 5 Gew.-% Polyäthylenglykol mit einem Molekulargewicht von 400 bis 2.000 enthält.

**Revendications**

1. Produit d'hygiéne dentaire à durée de conservation amèliorée eu égard à la rhéologie et au pourvoir abrasif, caractérisé par le fait qu'il contient en tant qu'agent épaississant et abrasif en quàntité de 20 à 25% en poids un acide silicique précipité qui est caractérisé par les données physico-chimiques suivantes:

| | |
|---|---|
| Surface BET selon DIN 66 131 | m²/g 15—110 |
| Densité apparente | g/l 90—650 |
| Courbe de distribution de taille de particules secondaires mesurées au compteur Coulter | selon la figure 2 |
| Résidu de tamis "ALPINE" >63 μm | % en poids <0,1 |
| Abrasion du Cu en dispersion à 10% dans le glycérol | mg 5—30 |
| Viscosité en dispersion à 30% dans un mélange eau-glycérol (1:1), Viscosimètre de Brookfield RTV, axe 5 | mPa 30 000—60 000 |
| Indice de luminosité relative selon DIN 55 921 | % 90—96 |

et qui peut être préparé par le fait que l'on dilue avec de l'eau chaude une suspension initiale d'acide silicique précipité, qui est préparée par précipitation de l'acide silicique dans une solution de silicate alcalin, préparée au préalable, à une concentration d'environ 5—25 g de SiO$_2$ par litre de solution, avec une solution de silicate de métal alcalin et d'acide, à des concentrations de solution déterminées et à des vitesses d'addition déterminées, en maintenant une température de précipitation dans le milieu réactionnel entre 80 et 90°C, de façon à maintenir relativement basse la viscosité du milieu réactionnel pendant une durée représentant au moins 30% de la durée totale de la précipitation, et le pH entre 10 et 12, et on arrête l'addition des corps en réaction dès que la viscosité, après être passée par un maximum, s'abaisse à une valeur inférieur à 100% au-dessus de la viscosité initiale, pour aboutit à une teneur en acide silicique précipité de 10—30 g/l et à une teneur en sulfate de sodium de 6—20 g/l, la chauffe à 85—95°C, ajuste le pH à 7—9 avec de l'acide sulfurique et, en maintenant ce pH constant par addition simultanée de solution de silicate de métal alcalin, d'acide sulfurique et d'eau chaude, ajuste en une durée de précipitation de 15—180 minutes une concentration finale d'acide silicique précipité allant de 40 à 60 g/l, acidifie la suspension à un pH inférieur à 7 avec de l'acide sulfurique concentré, sépare l'acide silicique précipité, hors de la suspension, à l'aide d'un filtre-presse, le lave, le sèche, puis le broie au moyen d'un broyeur désintégrateur.

2. Produit d'hygiène dentaire à durée de conservation amèliorée eu égard à la rhéologie et au pouvoir abrasif, caractérisé par le fait qu'il contient en tant qu'agent abrasif en quàntité de 20 à 25% en poids un acide silicique précipité qui est caractérisé par les données physico-chimiques suivantes:

| | |
|---|---|
| Surface BET selon DIN 66 131 | m²/g 15—110 |
| Densité apparente | g/l 150—750 |
| Distribution de tailles de particules secondaires | selon la figure 1 |
| Résidu de tamis "ALPINE" >63 μm | % en poids <1,5 |
| Abrasion du Cu en dispersion à 10% dans le glycérol | mg 5—30 |
| Viscosité en dispersion à 30% dans un mélange de-glycérol-eau (1:1), Viscosimètre de Brookfield RTV, axe 5 | mPa 30 000—60 000 |
| Indice de luminosité relative selon DIN 55 921 | % 86—96 |

et qui peut être préparé par le fait que l'on dilue avec de l'eau chaude une suspension initiale d'acide silicique précipité, qui est préparée par précipitation de l'acide silicique dans une solution de silicate alcalin, préparée au préalable, à une concentration d'environ 5—25 g de SiO$_2$ par litre de solution, avec une solution de silicate de métal alcalin et d'acide, à des concentrations de solution déterminées et à des vitesses d'addition déterminées, en maintenant une température de précipitation dans le milieu réactionnel entre 80 et 90°C, de façon à maintenir relativement basse la viscosité de milieu réactionnel pendant une durée représentant au moins 30% de la durée totale de la préparation, et le pH entre 10 et 12, et on arrête l'addition des corps en réaction des que la viscosité, après être passée par un maximum, s'abaisse à une valeur inférieure à 100% au-dessus de la viscosité initiale, pour aboutir à une teneur en acide silicique précipité de 10—30 g/l et à une teneur en sulfate de sodium de 6—20 g/l, la chauffe à 85—95°C, ajuste le pH à 7—9 avec de l'acide sulfurique et, en maintenant ce pH constant par addition simultanée de solution de silicate de métal alcalin, d'acide sulfurique et d'eau chaude, ajuste en une durée de précipitation de 15—180 minutes une concentration finale d'acide silicique précipité allant de 40 à 60 g/l, acidifie la suspension à un pH inférieur à 7 avec de l'acide sulfurique concentré, sépare l'acide silicique précipité, hors de la suspension, à l'aide d'un filtre-presse, le lave, le sèche, puis le broie au moyen d'un broyeur à cylindres centrifuges.

3. Produit d'hygiène dentaire selon les revendications 1 et 2, caractérisé par le fait qu'il contient en outre 0,5—5% en poids, de préférence 2 à 5% en poids de polyéthylèneglycol ayant un poids moléculaire de 400 à 2000.

**Claims**

1. A dentifrice with improved stability in storage with regard to rheology and abrasiveness, characterized in that it contains as thickening and abrasive agent in an amount of 20 to 25% by weight a precipitated silica which is characterized by the following physico-chemical data;

BET surface area according to DIN 66131: $m^2/g$ 15—110

Tamped density: g/l 90—650

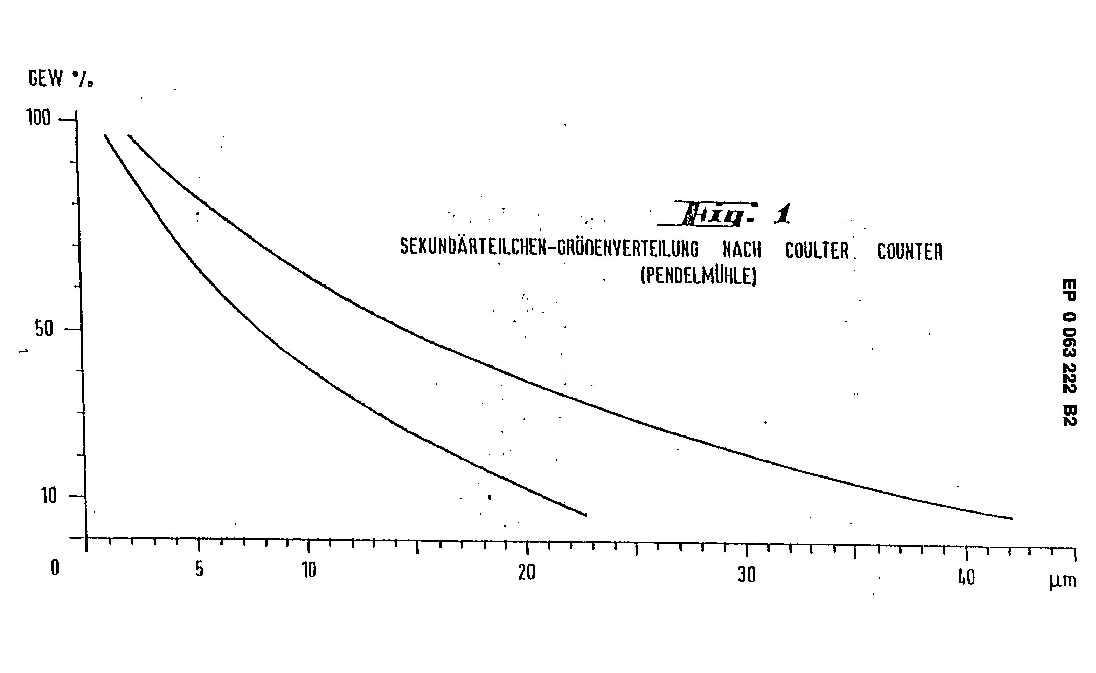

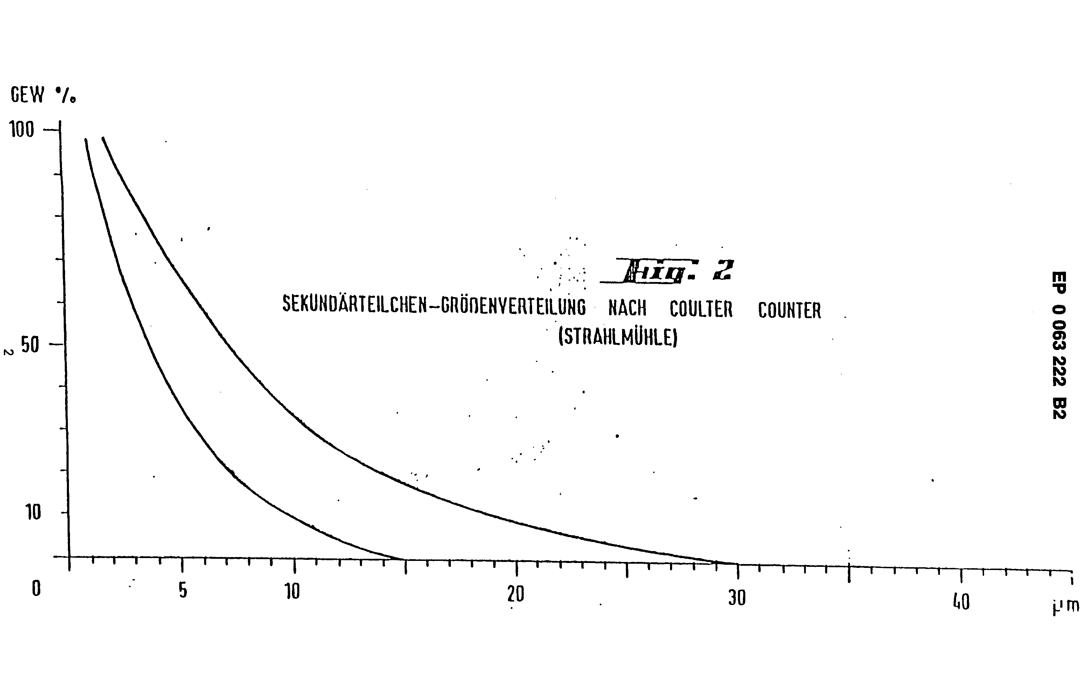

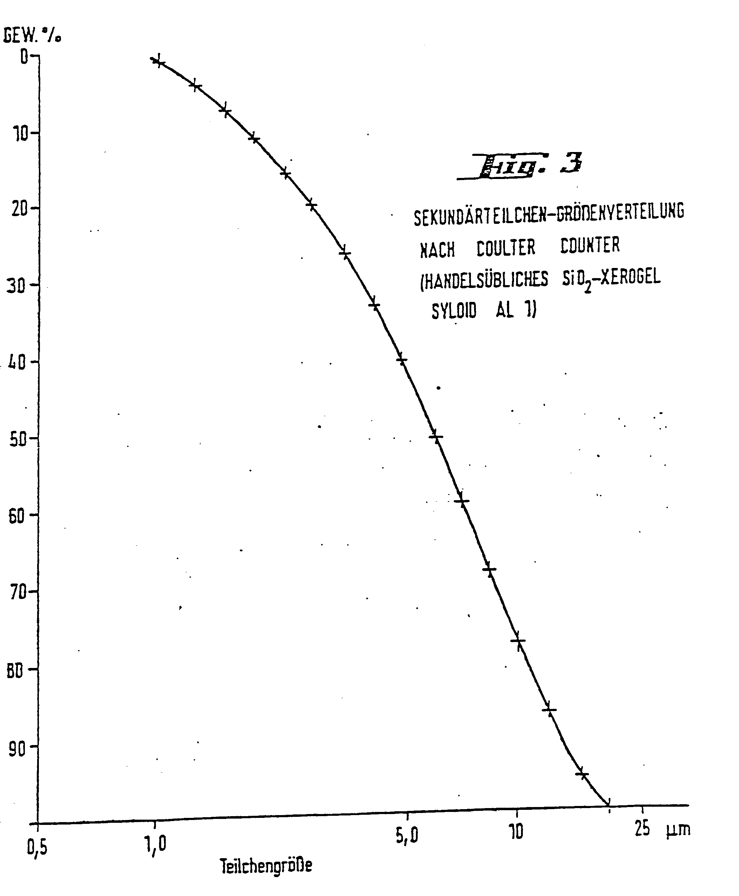

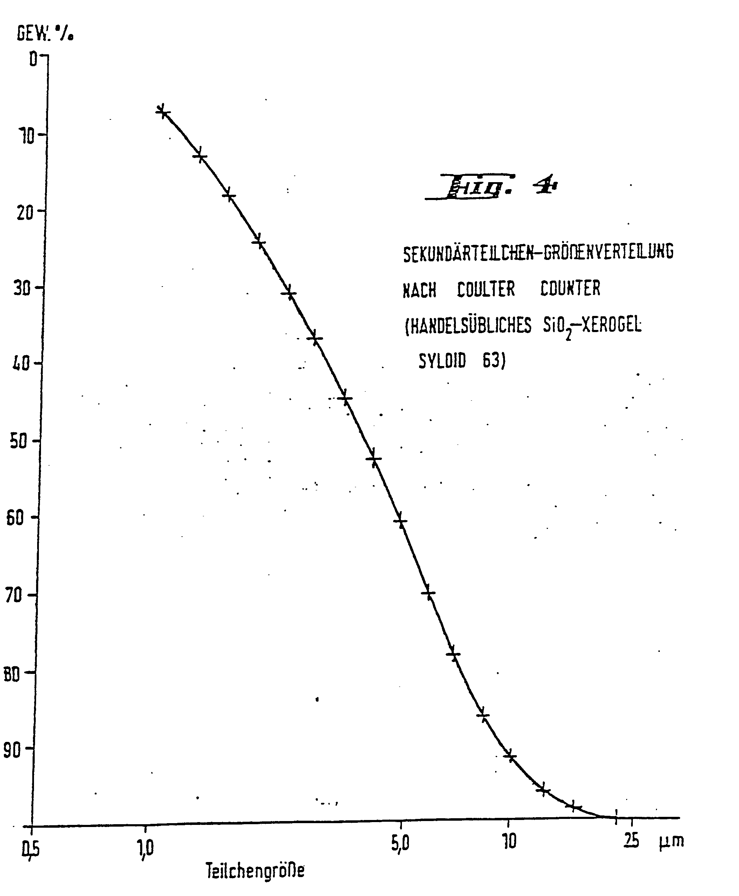

Secondary particle size distribution curve in Figure 2 according to Coulter Counter: "ALPINE" — Sieve residue >63 μm: % by weight <0.1

Cu-Abrasion in 10% glycerine dispersion: mg 5—30

Viscosity in 30% glycerine — water dispersion (1:1) Brookfield RTV Sp 5: mPas 30,000—60,000

Brightness reference value A according to DIN 55 921:% 90—96 and is obtainable in that an original precipitated silica suspension which is produced by precipitation of the silica from a starting alkali silicate solution having a concentration of about 5—25 g $SiO_2$ per litre of solution with an acid and alkali silicate solution having specific solution concentrations and specific supply rates while maintaining a precipitation temperature in the reaction medium of between 80 and 90°C in such a way that the viscosity of the reaction medium is kept uniformly low in a period of at least 30% of the total precipitation period and the pH is maintained between 10 and 12, and the addition of the reactants is ended only when the viscosity has passed through a maximum and fallen to a value lying lower than 100% above the starting viscosity, the mixture is diluted with hot water to a precipitated silica content of from 10 to 30 g/l and a sodium sulphate content of from 6 to 20 g of $Na_2SO_4/l$, is heated to between 85 and 95°C, the pH is adjusted to 7 to 9 with sulphuric acid and, while maintaining this pH by simultaneous supply of alkali silicate solution, sulphuric acid and hot water over a precipitation period of from 15 to 180 minutes, a final precipitated silica concentration of from 40 to 80 g/l is adjusted, the suspension is acidified to a pH below 7 with concentrated sulphuric acid, the precipitated silica is separated from the suspension using a filter press, is washed out, dried and ground by means of a jet mill.

2. A dentifrice with improved stability in storage with regard to rheology and abrasiveness, characterized in that it contains as abrasive agent in an amount of 20 to 25% by weight a precipitated silica which is characterized by the following physico-chemical data;

BET surface area according to DIN 66 131: $m^2/g$ 15—110

Tamped density: g/l 150—750

Secondary particle size distribution curve in Figure 1 according to Coulter Counter: "ALPINE" — Sieve residue >63 μm: % by weight <1.5

Cu-Abrasion in 10% glycerine dispersion: mg 5—30

Viscosity in 30% glycerine — water dispersion (1:1) Brookfield RTV Sp 5:m Pas 30,000—60,000

Brightness reference value A according to DIN 55 921:% 86—96 and is obtainable in that an original precipitated silica suspension which is produced by precipitation of the silica from a starting alkali silicate solution having a concentration of from about 5 to 25 g of $SiO_2$ per liter of solution with an acid and an alkali metal silicate solution having specific solution concentrations and specific supply rates while maintaining a precipitation temperature in the reaction medium of between 80 and 90°C in such a way that the viscosity of the reaction medium is kept uniformly low over a period of at least 30% of the total precipitation period and the pH is kept at between 10 and 12, and the addition of the reactants is ended only when the viscosity has passed through a maximum and has fallen to a value lying less than 100% over the starting viscosity, the mixture is diluted with hot water to a precipitated silica content of from 10 to 30 g/l and sodium sulphate content of from 6 to 20 g of $Na_2SO_4/l$, is heated to between 85 and 95°C, the pH is adjusted to between 7 and 9 using sulphuric acid and, while maintaining this pH by simultaneous supply of alkali metal silicate solution, sulphuric acid and optionally hot water over a precipitation period of from 15 to 180 minutes, a final precipitated silica concentration of from 40 to 80 g/l is adjusted, the suspension is acidified to a pH below 7 using concentrated sulphuric acid, the precipitated silica is separated from the suspension using a filter press, is washed out, dried and ground using a pendulum mill.

silica is separated from the suspension using a filter press, is washed out, dried and ground using a pendulum mill.

3. A dentifrice according to claims 1 to 2, characterized in that it also contains from 0.5 to 5% by weight, preferably from 2 to 5% by weight of polyethylene glycol having a molecular weight of from 400 to 2000.

GEW %

**Fig. 1**

SEKUNDÄRTEILCHEN-GRÖßENVERTEILUNG NACH COULTER COUNTER
(PENDELMÜHLE)

EP 0 063 222 B2

GEW %

**Fig. 2**

SEKUNDÄRTEILCHEN-GRÖßENVERTEILUNG NACH COULTER COUNTER
(STRAHLMÜHLE)

EP 0 063 222 B2

Fig. 3

SEKUNDÄRTEILCHEN-GRÖßENVERTEILUNG
NACH COULTER COUNTER
(HANDELSÜBLICHES $SiO_2$-XEROGEL
SYLOID AL 1)

Fig. 4

SEKUNDÄRTEILCHEN–GRÖßENVERTEILUNG
NACH COULTER COUNTER
(HANDELSÜBLICHES $SiO_2$–XEROGEL
SYLOID 63)